# EUROPÄISCHE PATENTSCHRIFT

(11) **EP 1 501 499 B1**
(45) Veröffentlichungstag und Bekanntmachung des Hinweises auf die Patenterteilung: **05.03.2008**
(21) Anmeldenummer: 03727432.1
(22) Anmeldetag: 05.05.2003
(51) Int. Cl.: A61K 31/381, A61K 31/045

(54) **TRANSEPIKUTANE DARREICHUNGSFORM ZUR BEHANDLUNG DES RESTLESS LEG SYNDROMS**
TRANS-EPICUTANEOUS ADMINISTRATION FORM FOR TREATING RESTLESS LEG SYNDROME
FORME GALENIQUE TRANSCUTANEE POUR LE TRAITEMENT DU SYNDROME DES JAMBES SANS REPOS

(30) Priorität: 06.05.2002 DE 10220230
(43) Veröffentlichungstag der Anmeldung: 02.02.2005
(73) Patentinhaber: SCHWARZ PHARMA AG, 40789 Monheim/Rhld. (DE)
(72) Erfinder: LAUTERBACH, Thomas, 40595 Düsseldorf (DE); SCHOLLMAYER, Erwin, 51375 Leverkusen (DE)
(74) Vertreter: HOFFMANN EITLE
(86) Internationale Anmeldenummer: PCT/EP2003/004685
(87) Internationale Veröffentlichungsnummer: WO 2003/092677

(56) Entgegenhaltungen:
- DE-A- 10 041 479
- FREEMAN ALAN ET AL: "Ropinirole for restless legs syndrome (RLS): An Open-Label and Double-Blind Placebo-Controlled Study." NEUROLOGY, Bd. 56, Nr. 8 Supplement 3, 24. April 2001 (2001-04-24), Seite A5 XP008020183 53rd Annual Meeting of the American Academy of Neurology;Philadelphia, PA, USA; May 05-11, 2001 ISSN: 0028-3878
- ZUCCONI M ET AL: "Effectiveness of the D2-agonist cabergoline as single-drug therapy for Restless Legs Syndrome: Clinical and actigraphic evaluation." SLEEP (ROCHESTER), Bd. 24, Nr. Abstract Supplement, 15. April 2001 (2001-04-15), Seite A19 XP008020184 15th Annual Meeting of the Associated Professional Sleep Societies;Chicago, Illinois, USA; June 05-10, 2001 ISSN: 0161-8105
- HUNDEMER H ET AL: "The safety of pergolide in the treatment of restless legs syndrome RLS: Results of a randomized long-term multicenter trial of pergolide in the treatment of RLS." SLEEP (ROCHESTER), Bd. 24, Nr. Abstract Supplement, 15. April 2001 (2001-04-15), Seite A17 XP008020186 15th Annual Meeting of the Associated Professional Sleep Societies;Chicago, Illinois, USA; June 05-10, 2001 ISSN: 0161-8105
- SILBER M H ET AL: "Pramipexole in the management of restless legs syndrome: An extended study." SLEEP (ROCHESTER), Bd. 24, Nr. Abstract Supplement, 15. April 2001 (2001-04-15), Seite A18 XP008020185 15th Annual Meeting of the Associated Professional Sleep Societies;Chicago, Illinois, USA; June 05-10, 2001 ISSN: 0161-8105
- METMAN LEO VERHAGEN ET AL: "Continuous transdermal dopaminergic stimulation in advanced Parkinson's disease." CLINICAL NEUROPHARMACOLOGY, Bd. 24, Nr. 3, Mai 2001 (2001-05), Seiten 163-169, XP008020182 ISSN: 0362-5664
- "Pschyrembel - Klinisches Wörterbuch, 260. Auflage" 2004, DE GRUYTER , BERLIN, NEW YORK * Seite 1755 *

## Beschreibung

Die Erfindung betrifft die Verwendung von Rotigotin zur Herstellung eines Arzneimittels in einer transepikutanen Applikationsform, insbesondere in Form eines Transdermalen Therapeutischen Systems (TDS) zur Behandlung des Restless Leg Syndroms.

### Hintergrund der Erfindung

Das Restless Leg Syndrom, nachfolgend auch mit RLS bezeichnet, ist eine neurologische Erkrankung und äußert sich als eine Mißempfindung in den Beinen, die mit starkem Bewegungsdrang einhergeht. RLS äußert sich durch Kribbeln, Ziehen, Reißen, Jucken, Brennen, Krämpfe oder Schmerzen und löst bei den Betroffenen den unwiderstehlichen Drang aus, sich zu bewegen. Diese Störungen treten gehäuft auf, wenn sich der jeweils Betroffene ausruht.

Besonders abends beim Einschlafen und während des nächtlichen Schlafes führen diese Gefühlsstörungen und der in Folge auftretende Bewegungsdrang zu Ruhelosigkeit und Schlafstörungen.

Das RLS kann in allen Altersstufen auftreten, jedoch nimmt die Häufigkeit im höheren Lebensalter zu. Die Prävalenz in der Allgemein-Bevölkerung liegt bei ca. 10 %. Aufgrund der Charakteristik der Symptome ist das RLS eine der häufigsten Ursachen von Schlafstörungen. Bei 20-40jährigen ist das RLS in 5 %, bei 40-60jährigen in 20 % und bei über 60jährigen in 35 % Ursache für Schlaf-Wachstörungen.

Wenn die Schlaf- bzw. Lebensqualität der Patienten zunehmend durch RLS eingeschränkt ist oder die Patienten an Tagesmüdigkeit leiden, ist die Indikation zur Therapie gegeben. Eine Therapiebedürftigkeit tritt in der Regel im Alter von 40-50 Jahren ein.

In Therapiestudien zeigten Monotherapien mit Dopaminagonisten, Opiaten, Benzodiazepinen, Carbamazepin, Clonidin oder Levodopa (L-DOPA) in Kombination mit einem Dopadecarboxylasehemmer unterschiedliche Erfolge.

Die Anwendung von L-DOPA bei RLS wurde am häufigsten untersucht. Bei der Langzeittherapie mit L-DOPA kommt es zu einer deutlichen Beschwerdeabnahme mit Verbesserung der Lebens- und Schlafqualität. Der Nachteil der Therapie besteht jedoch darin, daß bei einer Vielzahl von Patienten die Wirkung wegen der kurzen Halbwertszeit von L-DOPA nur kurz anhält. Außerdem kommt es bei der Langzeitanwendung zu einer nachlassenden Wirkung (Toleranzentwicklung) und/oder zu einer Verschiebung der RLS-Beschwerden in die Morgenstunden (Rebound) oder einer Verschlimmerung der Beschwerden mit einem Auftreten von Störungen auch über Tag (Augmentation).

Augmentation, Rebound und Toleranz werden bei dem RLS-Leiden im einzelnen, wie folgt, beschrieben:
a) Augmentation
   Bei RLS-Patienten umfasst die Augmentation
   - einen früheren Eintritt der RLS-Symptome am Abend als vor der Behandlung;
   - Auftreten der Symptome während des Tages;
   - Betroffenheit anderer Körperteile, typischerweise der Arme;
   oder
   - eine raschere Progression der Symptome als bei unbehandeltem Verlauf.

   Besonders eine Form der Augmentation bei RLS-Patienten ist unerwünscht. Sie wird beschrieben mit einer Steigerung der Schwere der RLS-Beschwerden tagsüber, gefolgt von einer Abnahme der Symptome nachts, wenn die Einnahme des Medikaments jeweils abends erfolgt.
b) Rebound
   Dieses Phänomen ist der Augmentation ähnlich und äußert sich insbesondere im Auftreten der RLS-Symptome in den Morgenstunden, kurz nach dem Aufwachen bei abklingendem Wirkspiegel.
c) Toleranz
   Sie kann bei RLS-Patienten als Gewöhnung an eine wirkstoffbasierte Therapie beschrieben werden.
   Sie äußert sich dadurch, dass entweder immer höhere Wirkstoffdosierungen an den Patienten verabreicht werden müssen, um dieselben Symptome des RLS-Leidens zu lindern, oder dass die gleiche Wirkstoffmenge weniger Linderung der Beschwerden bewirkt, als dies zu Beginn der Therapie der Fall war.

Weitere einzelne Dopaminagonisten wurden in Kurzzeittherapie-Studien auf ihre therapeutische Verwendbarkeit geprüft. Zu den untersuchten Dopaminagonisten zählen: Bromocriptin, Cabergolin, Alpha-Dihydroergocryptin, Lisurid, Pergolid, Pramipexol und Ropinirol. Es zeigte sich, daß alle diese Dopaminagonisten wirksam sind, jedoch den Nachteil aufweisen, Nebenwirkungen wie Übelkeit, Erbrechen, Schwindel, Hypotonie, Obstipation, Schlaflosigkeit, die in der Regel initial und dosisabhängig auftreten, auszulösen.

Benzodiazepine und Opiate werden ebenfalls bei RLS eingesetzt. Wegen der Gefahr der Abhängigkeit und der Toleranzentwicklung stehen diese Substanzen jedoch nur eingeschränkt für eine Therapie zur Verfügung.

Auch die Wirkung von transdermal verabreichtem Clonidin, 2-(2,6-Dichloranilino)-4,5-dihydroimidazol, das ursprünglich als Antihypertonikum und Miotikum entwickelt wurde, wurde bei der Behandlung von RLS untersucht. Dabei wurde festgestellt, daß zwar die Einschlaflatenz verkürzt, die Schlafqualität, Häufigkeit des Aufwachens oder die periodischen Beinbewegungen im Schlaf (Periodic Leg Movements during Sleep, "PLMS") dagegen nicht beeinflußt wurden. Da als Monotherapie wirksamere Substanzen zur Verfügung stehen, wird derzeit Clonidin als alternative Therapieform nur bedingt empfohlen.

Die meisten bisherigen Monotherapien, beispielsweise die Therapie mit L-Dopa, weisen den Nachteil auf, daß die Menge des entsprechenden Wirkstoffs in Abhängigkeit der Therapiedauer gesteigert werden muß, um den therapeutischen Erfolg zu sichern. Eine Therapie ist somit notwendigerweise mit zunehmender Wirkstofftoleranz verbunden.

Daher sollen Kombinationstherapien die mit den Monotherapien verbundenen Nachteile überwinden.

So beschreibt WO 01/13903 eine unter anderem in einem TDS enthaltene Wirkstoffkombination zur Behandlung des Restless Leg Syndroms, bestehend aus einem α2-Agonist und einem weiteren in Monotherapie die Symptome von RLS reduzierenden Neuropsychopharmakon.

Unter anderem wird als Neuropsychopharmakon aus der Gruppe der Dopaminagonisten S(-)-2-(N-propyl-N-2-thienylethylamino)-5-hydroxy-tetralin (z.B. als N-0923) als ein Bestandteil der Wirkstoffkombination erwähnt. Ausführungsbeispiele hierzu fehlen jedoch.

WO 01/13902 definiert eine Wirkstoffkombination unter anderem zur transdermalen Applikation, bestehend aus dem α2-Agonist Clonidin und dem Dopaminagonisten Pramipexol zur Behandlung des Restless Leg Syndroms.

In beiden Dokumenten erläutern die Ausführungsbeispiele die Behandlung zweier Patienten mit einer Kombinationstherapie bestehend aus Pramipexol und Clonidin.

Pramipexol ist grundsätzlich zur Behandlung der sensomotorischen RLS-Symptome anerkannt.

Als beachtliche, weitere, unerwünschte Wirkungen (Nebenwirkungen) dieser Substanz werden Müdigkeit, gestörte Verdauung (Dyspepsie), Kopfschmerz und Sekretionsstau berichtet.

Da das in der Wirkstoffkombination vorhandene Clonidin - wie in den WO-Schriften vorgeschlagen - jedoch keinen Einfluss auf die Schlafqualität, die Häufigkeit des Aufwachens oder die periodischen Beinbewegungen im Schlaf hat, leistet somit der Wirkstoff Clonidin keinen wesentlichen Beitrag zur Linderung der Krankheit RLS.

Wie bereits erwähnt, ist daher der α2-Agonist Clonidin bei der Behandlung der RLS-Krankheit nur die 2. Wahl.

Dies bestätigt die U.S. amerikanische Restless Leg Syndrome Foundation in ihrem "RLS Medical Bulletin".

Im Kapitel "Treatment" (Behandlung) wird nach "Primary Pharmacological Treatments" auf "Secondary Pharmacological Treatments" eingegangen. "Secondary Pharmacological Treatments" sind dort als nicht gut etablierte Behandlungen oder solche mit begrenzter Effektivität bei der Behandlung von RLS qualifiziert.

Neben anderen Wirkstoffen wird auch Clonidin bei der RLS-Krankheit als nicht hinreichend effektiv wirkend erwähnt.

Es wird die Schlußfolgerung gezogen, dass die Verwendung von Clonidin bei der Behandlung von RLS nicht mit Überzeugung empfohlen werden kann. Die Patienten müssten verstehen, dass der Beweis für einen Nutzen dieser Substanz minimal ist.

Schließlich wurde die in den beiden vorgenannten WO-Schriften beschriebene Behandlung mit der Kombinationstherapie nur an insgesamt 2 Probanden (jeweils ein (1) männlicher und ein (1) weiblicher) durchgeführt, so dass keine Aussage über die Therapie und deren Verlauf möglich ist.

Auch sind die vorgeschlagenen Pflasterformulierungen entweder umständlich zu handhaben, technisch schwierig zu verwirklichen oder wirtschaftlich kostenintensiv.

Werden, wie in den vorgenannten WO-Schriften ausgeführt, die beiden Wirkstoffe in je einem separaten Pflaster gegeben, ist die Handhabung für den Patienten umständlich und eine hinreichende Compliance nicht gegeben. Es besteht u.a. die Gefahr, dass die Pflaster verwechselt werden und daher vom Patienten zwei (2) wirkstoffgleiche Pflaster appliziert werden.

Werden die beiden Wirkstoffe in einem gemeinsamen Pflaster als Gemisch gelagert, ist nicht sichergestellt, dass die therapeutisch erforderliche Dosis jeder Einzelkomponente zur Wirkung kommen kann. Somit ist die erforderliche Wirksamkeit nicht hinreichend gewährleistet.

Werden die beiden Wirkstoffe innerhalb des Pflasters getrennt gelagert, so ist die Herstellung eines solchen Pflasters konstruktiv aufwendig und kostenintensiv.

Schließlich sollte die Therapie einer Krankheit möglichst mit Einzelsubstanzen erfolgen, um Wechselwirkungen mit anderen verabreichten Substanzen so gering wie möglich zu gestalten. Diese therapeutische Forderung ist bei der RLS-Krankheit von Bedeutung, da die Krankheit - wie eingangs erwähnt - in fortgeschrittenem Alter auftritt, in dem Multimorbidität häufig auftritt.

Bei der RLS-Medikation sind eine Reihe von Hindernissen zu überwinden.

Beispielsweise ist bei der Verabreichung von Levodopa lediglich eine begrenzte und unregelmäßige Absorption im Darm, insbesondere Zwölffingerdarm möglich, so dass keine reproduzierbaren Plasmaspiegel erreicht werden. Dies gilt insbesondere bei gleichzeitiger Nahrungsaufnahme.

Die meisten bei der Krankheit RLS einsetzbaren Wirkstoffe unterliegen bei oraler Verabreichung einem First-Pass-Metabolismus der Leber.

Diese Faktoren können bei oraler Verabreichung einen verzögerten und nicht reproduzierbaren Wirkungseintritt und eine nicht vorhersehbare Wirkdauer zur Folge haben. Insbesondere sind die verursachten fluktuierenden Levodopa-Plasma Konzentrationen unerwünscht. Die Spitzenwerte der Plasmakonzentrationen korrelieren oft mit dem Auftreten unerwünschter Nebenwirkungen, bei zu niedrigem Plasmaspiegel läßt hingegen die Wirkung nach. L-Dopa muß deshalb mehrmals täglich gegeben werden, was zu Compliance Problemen führen kann.

Die vorgenannten Nachteile der oralen Verabreichung führten dazu, andere, wirkungsvollere Verabreichungswege für Levodopa oder andere Wirkstoffe gegen die RLS-Krankheit zu suchen. Die folgenden nicht-oralen Verabreichungswege wurden in Betracht gezogen: intravenös, transdermal, subcutan, intramuskulär, intracerebroventrikulär, intranasal, pulmonal, sublingual oder intrarectal.

Im Stand der Technik sind bisher verschiedenste Dopamin-Agonisten zur Behandlung des RLS-Syndroms beschrieben, die jedoch oral verabreicht wurden und in der Folge zu den bekannten Augmentations- und Rebound Phänomenen führten und mit unangenehmen Nebenwirkungen verbunden waren. So wird die orale Verabreichung von Ropinirol (A. Freemnan et al., Neurology, Bd. 56, Nr. 8 Supplement 3, 2001, S. A5), Cabergolin (M. Zucconi et al., SLEEP, Bd. 24, 2001, S. A19), Pergolide (H. Hundermer et al., SLEEP, Bd. 24, 2001, S. A17) in Kurzzeitstudien und Pramipexol (M. Silber et al., SLEEP, Bd. 24, 2001, S. A18) in einer Langzeitstudie beschrieben. Alle angegebenen Dopamin-Agonisten führten zu den beschriebenen Augmentations- und Rebound Phänomenen und damit verbundenen Nebenwirkungen.

L.V. Metman et al. (Clinical Neuropharmacology, Bd. 24, Nr. 3, 2001, S. 163-169) beschreibt die transdermale Verabreichung von Rotigotin zur Behandlung des Parkinson-Syndroms.

Jedoch führte bisher keiner dieser Wege zu therapeutisch befriedigenden Verabreichungssystemen oder Therapieerfolgen.

Insbesondere zeigen die vorgeschlagenen nicht-oralen Verabreichungswege die Beschränkungen, dass sie zum Teil invasiv sind und Komplikationen auftreten können. Dies war z.B. bei Langzeit-Intraduodenal-Verabreichung einer Levodopa-Lösung der Fall. Insbesondere ist der Austausch der Katheterspritzen häufig mit Wunden und Schmerz verbunden.

Auch die kontinuierliche intravenöse oder subkutane Infusion ist therapeutisch nicht zufriedenstellend. Zwar können z.B. mit subkutan verabreichten Infusionen von Apomorphin stabile Plasmakonzentrationen erreicht werden, jedoch treten nicht akzeptable lokale Irritationen auf, wenn Apomorphin mehrmals täglich über einen längeren Zeitraum verabreicht werden muss.

Daher bestand und besteht weiterhin Bedarf, einen Wirkstoff für die Therapie der Krankheit RLS zur Verfügung zu stellen,
■ der ausgezeichnet verträglich ist,
■ monotherapiert werden kann,
■ in einer pharmazeutisch-technischen Formulierung vorliegt, die die therapeutischen Anforderungen erfüllt,
und
mit dem die wirkstoffhaltige Formulierung sowohl technisch als auch unter Kostenaspekten günstig gefertigt werden kann.

Überraschenderweise und unerwartet wurde gefunden, dass eine Rotigotin enthaltende transepikutane Darreichungsform, insbesondere in Form eines TDS zur Behandlung des RLS therapeutisch wertvoll ist. Insbesondere für die Behandlung von RLS-Patienten, die auf eine kontinuierliche Dauerbehandlung der RLS-Symptome angewiesen und für RLS-Augmentation anfällig sind, ist diese Darreichungsform therapeutisch effektiv.

### Beschreibung der Erfindung

Die vorliegende Erfindung betrifft daher die Verwendung von Rotigotin zur Herstellung einer pharmazeutischen Zusammensetzung in Form einer transepikutanen pharmazeutischen Zubereitung, insbesondere in Form eines Transdermalen Therapeutischen Systems (TDS), das die aus dem Stand der Technik bekannten Nachteile bisheriger Monotherapien mit oral verabreichten Wirkstoffen vermeidet.

Überraschenderweise hat sich gezeigt, daß die Gabe von Rotigotin als Monotherapeutikum in einer transepikutanen Zusammensetzung, insbesondere in Form der pharmazeutischen Zusammensetzung eines TDS die Suppression und Reduktion der RLS-Symptome bewirkt, wobei der Wirkstoff Rotigotin im Vergleich zu bisher bekannten Monotherapien auch dauerhaft sehr niedrig dosiert werden kann und gut vertragen wird.

Die erfindungsgemäße Darreichungsform eignet sich besonders für die Behandlung mittlerer bis schwerer Erkrankungen von RLS.

Rotigotin ist der INN (International Nonproprietary Name) der chemischen Substanz (-)-5,6,7,8-Tetrahydro-6-[propyl[2-(2-thienyl)ethyl]amino]-1-naphtol.

Die Verbindung (-)-5,6,7,8-Tetrahydro-6-[propyl[2-(2-thienyl)ethyl]amino]-1-naphtol wird in an sich bekannter Weise erhalten. Die Herstellung der vorgenannten Verbindung wird, wie in der EP 0 168 505 B1 beschrieben, hergestellt. Hierauf wird für die vorliegende Erfindung vollumfänglich Bezug genommen.

Es ist bereits bekannt, daß (-)-5,6,7,8-Tetrahydro-6-[propyl[2-(2-thienyl)ethyl]amino]-1-naphtol als Mittel zur Behandlung des Parkinson-Syndroms verwendet werden kann.

Jedoch ist in diesem Zusammenhang der Hinweis erheblich, dass die Krankheit RLS keine Form der Parkinson'schen Krankheit, sondern vielmehr ein von dieser verschiedenes Leiden ist.

Es wurde nun gefunden, daß die Verabreichung von (-)-5,6,7,8-Tetrahydro-6-[propyl[2-(2-thienyl)ethyl]amino]-1-naphtol in einer transepikutanen Darreichungsform die Erkrankung Restless Leg Syndrom vorteilhaft beeinflußt.

Überraschenderweise zeigt die erfindungsgemäß zu verwendende Verbindung bereits in sehr niedrigen Dosen eine spezifische Wirkung, die ihre Verwendung bei der Behandlung des Restless Leg Syndroms ermöglicht. Sowohl in der Wirkungsstärke als auch in ihrer Spezifität ist die Verbindung den bekannten in der Indikation des Restless Leg Syndroms wirksamen Stoffen überlegen.

In der Reihe der aus dem Stand der Technik bekannten Monotherapeutika gegen RLS ist bisher eine derartig effiziente und spezifische Wirkung nicht bekannt geworden, so daß die Verwendung der Verbindung in der erfindungsgemäßen Darreichungsform als Therapeutikum des Restless Leg Syndroms eine Bereicherung der Pharmazie und für den Arzt darstellt.

Die erfindungsgemäße Darreichungsform stellt in therapeutisch vorteilhafter Weise konstante Plasmaspiegel zur Verfügung, die die bei oralen Verabreichungsformen auftretenden pulsatilen Plasmaspiegel vermeidet. Insbesondere ist eine Food-Interaction nicht gegeben, wie sie von der L-Dopa Therapie mit der Folge, daß die Plasmaspiegel und therapeutischen Wirkungen nicht reproduzierbar sind, bekannt ist.

Ein Vorteil der Erfindung liegt darin, daß bei Verwendung des Wirkstoffs Rotigotine in einer transdermalen Applikationsform zur Behandlung des Restless Leg Syndroms bereits niedrige Dosen ausreichen, um das Befinden des Patienten zu verbessern, ohne daß dabei intolerable unerwünschte Wirkungen (Nebenwirkungen) auftreten; insbesondere ist erheblich, daß eine Augmentation supprimiert wird. Dies ist besonders wünschenswert, da bei ungefähr der Hälfte der RLS-Patienten mit Augmentation ein Medikamentationswechsel erforderlich ist. Darüber hinaus wird die Ansprechbarkeit und die Responderrate bei den RLS-Patienten verbessert. Besonders vorteilhaft im Sinne der Erfindung wird der Wirkstoff in der Menge von 1,0 bis 10 mg, bevorzugt in der Menge aus 0,5 bis 5 mg pro Tag appliziert.

Ein weiterer Vorteil der erfindungsgemäßen Darreichungsform ist in der bequemen Zurverfügungstellung des Wirkstoffes Rotigotin über mindestens 24 h mit konstanten Plasmaspiegeln begründet.

Der Wirkstoff wird in Form einer transepikutanen Applikation entweder als Salbe, Gel oder Creme auf die Haut des Patienten aufgetragen, bevorzugt wird er jedoch als TDS in Form eines Pflasters verabreicht.

Erfindungsgemäß enthält das Transdermale Therapeutische System eine gegenüber den Inhaltsstoffen der Matrix inerte Rückschicht, eine (-)-5,6,7,8-Tetrahydro-6-[propyl[2-(2-thienyl)ethyl]amino]-1-naphtol enthaltende, selbstklebende Matrixschicht und eine vor Gebrauch zu entfernende Schutzfolie, die dadurch gekennzeichnet ist, daß die Matrixschicht
a) als Basis einen nicht-wäßrigen Polymerkleber auf Acrylat- bzw. Silikonbasis enthält,
b) eine Löslichkeit für die freie Base (-)-5,6,7,8-Tetrahydro-6-[propyl[2-(2-thienyl)ethyl]amino]-1-naphtol von ≥ 5 % (g/g) besitzt, und
c) die freie Base (-)-5,6,7,8-Tetrahydro-6-[propyl[2-(2-thienyl)ethyl]amino]-1-naphtol in einer wirksamen Menge enthält.

Nach einer Weiterbildung enthält die Matrix des TDS < 0,5 % (g/g) anorganische Silikatpartikel.

Nach einer besonders vorteilhaften Weiterbildung enthält das Transdermale Therapeutische System < 0,05 % (g/g) anorganische Silikatpartikel in der Matrix.

Nach einer Ausführungsform der Erfindung enthält das Transdermale System einen Polymerkleber auf Acrylatbasis, der wenigstens zwei der folgenden Monomere enthält:
Acrylsäure, Acrylamid, Hexylacrylat, 2-Ethylhexyl-acrylat, Hydroxyethylacrylat, Octylacrylat, Butylacrylat, Methylacrylat, Glycidylacrylat, Methacrylatsäure, Methacrylamid, Hexylmethylacrylat, 2-Ethylhexylmethacrylat, Octylmethacrylat, Methylmethacrylat, Glycidylmethacrylat, Vinylacetat oder Vinylpyrrolidon.

Nach einer vorteilhaften Weiterbildung der Erfindung enthält das Transdermale System einen Polymerkleber auf Silikonbasis mit Zusatzstoffen zur Verbesserung der Löslichkeit von (-)-5,6,7,8-Tetrahydro-6-[propyl[2-(2-thienyl)ethyl]amino]-1-naphtol in Form von hydrophilen Polymeren oder Glycerin oder Glycerinderivaten.

Nach einer anderen erfindungsgemäßen Ausführungsform ist in dem Transdermalen System (-)-5,6,7,8-Tetrahydro-6-[propyl[2-(2-thienyl)ethyl]amino]-1-naphtol in dem Polymerkleber auf Acrylatbasis in einer Konzentration von 10 bis 35 % [g/g] oder in dem Polymerkleber auf Silikonbasis in einer Konzentration von 5 bis 40 % [g/g] enthalten.

Nach einer weiteren Weiterbildung der Erfindung enthält das Transdermale System Substanzen, die die Permeation von (-)-5,6,7,8-Tetrahydro-6-[propyl[2-(2-thienyl)ethyl]amino]-1-naphtol in die menschliche Haut verbessern.

Erfindungsgemäß ist für das Transdermale System die permeationsfördernde Substanz aus der Gruppe der Fettalkohole, Fettsäuren, Fettsäureester, Fettsäureamide, Glycerin oder seinen Derivaten, N-Methylpyrrolidon, Terpenen oder Terpenderivaten ausgewählt.

Nach einer erfindungsgemäßen Ausführungsform ist in dem Transdermalen Therapeutischen System die permeationsfördernde Substanz Ölsäure oder Oleylalkohol.

Erfindungsgemäß vorteilhaft ist in dem Transdermalen System das hydrophile Polymer Polyvinylpyrrolidon, ein Copolymer von Vinylpyrrolidon und Vinylacetat, Polyethylenglykol, Polypropylenglykol oder ein Copolymer von Ethylen und Vinylacetat.

In einer weiteren Ausführungsform ist in dem Transdermalen System das hydrophile Polymer in Form löslichen Polyvinylpyrrolidons und in einer Konzentration von 1,5-5 % (g/g) in der wirkstoffhaltigen Matrixschicht enthalten.

Weiterhin kann erfindungsgemäß das Transdermale System in der Matrix inerte Füllstoffe zur Verbesserung der Kohäsion enthalten.

Das Transdermale Therapeutische System kann, wie in den Ausführungsbeispielen der EP 1 033 978 B1 ausführlich beschrieben, hergestellt werden.

Ein pharmazeutisches Produkt gemäß der vorliegenden Erfindung umfaßt eine Verstärkungsschicht, die hinsichtlich der Bestandteile der Matrix inert ist, eine selbstklebende Matrixschicht, die eine wirksame Rotigotin- oder Rotigotinhydrochlorid-Menge umfaßt, und einen Schutzfilm, der vor der Verwendung zu entfernen ist.

### Ausführungsbeispiel 1

### Polyacrylatsystem mit (-)-5,6,7,8-Tetrahydro-6-[propyl [2-(2-thienyl)-ethyl]-amino]-1-naphtol

Zu 264 g einer Lösung eines Polyacrylatklebers mit einem Feststoffgehalt von 50 % werden 66 g einer 50 %igen Lösung von Eudragit E100 in Ethylacetat gegeben und nach Zugabe von 36 g Oleylalkohol die Masse durch Rühren homogenisiert.

Danach werden 89,65 g (-)-5,6,7,8-Tetrahydro-6-[propyl[2-(2-thienyl)ethyl]amino]-1-naphthol in 200 ml Methylethylketon gelöst und obiger Masse unter Rühren zugegeben. Nachdem die Masse homogenisiert ist, wird sie mit einer geeigneten Rakel auf eine silikonisierte Polyesterfolie beschichtet. Die Dicke des feuchten Films ist so bemessen, daß nach dem Entfernen der Lösemittel durch 30-minütiges Trocknen bei 50°C ein Beschichtungsgewicht von 60 g/m2 resultiert.

Der getrocknete Matrixfilm wird nun mit einer 13 µm dicken Polyesterfolie kaschiert. Aus dem resultierenden Pflasterlaminat werden nun die fertigen Pflaster in der gewünschten Größe ausgestanzt und in Packstoffbeutel verpackt.

Die Konzentration von (-)-5,6,7,8-Tetrahydro-6-[propyl[2-(2-thienyl)ethyl]amino]-1-naphthol in der Pflastermatrix ist 30,8 %. Geeignete Polyacrylatkleber sind z.B. Durotak 387-2051, Durotak 387-2287, Durotak 387-2353, Durotak 387-2516, alle von National Starch & Chemical.

### Ausführungsbeispiel 2

### Silikonsystem mit (-)-5,6,7,8-Tetrahydro-6-[propyl[2-(2-thienyl)-ethyl]-amino]-1-naphtol

Zu 24 g einer 25 prozentigen Lösung von Kollidon 90F werden 18 g (-)-5,6,7,8-Tetrahydro-6-[propyl[2-(2-thienyl)ethyl]amino]-1-naphthol gelöst in 40 g Ethanol zugegeben und die Masse homogenisiert. Zu dieser Masse werden anschließend 251 g einer Lösung eines aminresistenten Silikonklebers mit einem Feststoffgehalt von 70 % gegeben und die Masse durch weiteres Rühren homogenisiert.

Anschließend wird die Masse mit einer geeigneten Rakel auf eine adhäsiv ausgerüstete Polyesterfolie (Scotchpak 1022) in der Dicke beschichtet, daß nach dem Entfernen der Lösemittel durch 30-minütiges Trocknen bei 50 °C ein Beschichtungsgewicht von 50 g/m² resultiert.

Der getrocknete Matrixfilm wird nun mit einer 13 µm dicken Polyesterfolie kaschiert. Aus dem resultierenden Pflasterlaminat werden die fertigen Pflaster in der gewünschten Größe ausgestanzt und in Packstoffbeutel verpackt.

Die Konzentration von (-)-5,6,7,8-Tetrahydro-6-[propyl[2-(2-thienyl)ethyl]amino]-l-naphthol Base in der Pflastermatrix ist 9 %.

Geeignete aminresistente Silikonkleber sind z.B. BIO-PSA Q7-4301 und BIO-PSA Q7-4201, beide von Dow Corning.

### Ausführungsbeispiel 3

25 g (-)-5,6,7,8-Tetrahydro-6-[propyl[2-(2-thienyl)ethyl]amino]-1-naphthol-hydrochlorid werden zusammen mit 14,7 g Natriummetasilikat oder 16,8 g Natriumtrisilikat in 40 ml Ethanol über 48 Stunden bei Raumtemperatur gerührt. Optional wird die Wirkstofflösung nun filtriert und 9,2 g Oleylalkohol, 63,2 g einer 52 %igen Lösung eines Polyacrylatklebers (Durotak 387-2287, Fa. National Starch & Chemical) und 22,8 g einer 40 % (g/g) Lösung von Eudragit E100 (Röhm-Pharma) zugegeben und die Masse anschließend durch mechanisches Rühren homogenisiert.

Anschließend wird die Masse zur Herstellung der Pflastermatrix auf eine geeignete adhäsiv ausgerüstete Folie beschichtet und die Lösemittel durch 20minütiges Trocknen bei 50°C entfernt. Das Beschichtungsgewicht des getrockneten Matrixfilms liegt bei 80 g/m².

Der getrocknete Matrixfilm wird mit einer 23 µm dicken Polyesterfolie kaschiert. Aus dem Gesamtlaminat werden die einzelnen Pflaster gestanzt.

Schließlich ist erfindungsgemäß ganz besonders vorteilhaft, ein transdermales therapeutisches System auf Silikonbasis zu verwenden, das mindestens eine gegenüber Amin beständige Silikonverbindung als Hauptkomponente enthalten muß.

Gewöhnlich ist die Silikonverbindung ein auf Druck reagierendes Haftmittel oder eine Mischung davon und formt eine Matrix, in die andere Komponenten des TDS eingebettet werden. Das Haftmittel/die Haftmittel sollten darüber hinaus bevorzugt pharmazeutisch annehmbar sein, so dass sie biokompatibel, nicht sensitisierend und nicht reizend gegenüber der Haut sind. Besonders vorteilhafte Silikonhaftmittel zur erfindungsgemäßen Verwendung sollten außerdem die folgenden Erfordernisse erfüllen.
- bleibende Haft- und kohäsive Eigenschaften bei Vorhandensein von Feuchtigkeit oder Schweiß bei normalen Temperaturschwankungen,
- gute Kompatibilität mit Rotigotin sowie mit den anderen in der Formulierung verwendeten Trägern, insbesondere sollte das Haftmittel nicht mit der Amingruppe von Rotigotin reagieren.

Es wurde gezeigt, dass auf Druck reagierende Haftmittel vom Typ, der ein lösliches polykondensiertes Polydimethylsiloxan (PDMS)/Harznetzwerk bildet, wobei die Hydroxy-Endgruppen mit zum Beispiel Trimethylsilyl (TMS) Gruppen abgedeckt sind, erfindungsgemäß besonders nützlich sind. Bevorzugte Haftmittel dieser Art sind auf Druck reagierende BIO-PSA Silikonhaftmittel, die von Dow Corning hergestellt werden, insbesondere die Qualitäten Q7-4201 und Q7-4301. Andere Silikonhaftmittel können jedoch gleichermaßen verwendet werden.

Des weiteren und bevorzugt wird erfindungsgemäß für die gleiche Verwendung außerdem ein Transdermales Therapeutisches System auf Silikonbasis bereitgestellt, das zwei oder mehrere Silikonharze als klebende Hauptkomponenten umfasst. Es kann von Vorteil sein, wenn eine solche Mischung aus Silikonhaftmitteln mindestens ein stark klebendes Haftmittel und mindestens ein Haftmittel mit mittlerem Klebevermögen umfasst, um ein optimales Gleichgewicht zwischen guter Haftung und wenig kaltem Fluss zu gewährleisten. Übermäßiger kalter Fluss kann zu einem zu weichen Pflaster führen, das leicht an der Packung oder an der Kleidung des Patienten haftet. Darüber hinaus scheint eine solche Mischung von Haftmitteln besonders nützlich zu sein, um ein wirksames Transdermales Therapeutisches System zu erhalten. Eine Mischung aus den zuvor genannten gegenüber Amin beständigen, auf Druck reagierenden Silikonhaftmitteln Q7-4201 (mittleres Haftvermögen) und Q7-4301 (stark haftend) in ungefähr gleichen Mengen erwies sich erfindungsgemäß als besonders nützlich.

In einer weiteren bevorzugten Ausführungsform umfasst das transdermale therapeutische System auf Silikonbasis einen Lösungsvermittler. Verschiedene Tensid-ähnliche oder amphiphile Substanzen können als Lösungsvermittler verwendet werden. Sie sollten pharmazeutisch annehmbar und für die Verwendung bei Medikamenten zugelassen sein. Es ist von Vorteil, wenn der Lösungsvermittler auch dahingehend wirkt, dass die Kohäsion des Transdermalen Therapeutischen Systems verbessert wird. Ein besonders bevorzgutes Beispiel eines solchen Lösungsvermittlers ist lösliches Polyvinylpyrrolidon. Polyvinylpyrrolidon ist im Handel erhältlich, zum Beispiel unter dem Markennamen Kollidon^{®} (Bayer AG). Andere Beispiele umfassen Copolymere aus Polyvinylpyrrolidon und Vinylacetat, Polyethylenglycol, Polypropylenglycol, Glycerin und Fettsäureester aus Glycerin oder Copolymere aus Ethylen und Vinylacetat.

Das Transdermale Therapeutische System auf Silikonbasis enthält zur erfindungsgemäßen Verwendung weniger als 1 Gew.-% anorganischer Silikate, am meisten bevorzugt ist es vollständig frei von anorganischen Silikaten.

Der Wassergehalt des transdermalen therapeutischen Systems zur erfindungsgemäßen Verwendung ist bevorzugt so niedrig, dass keine Wasserverdampfung während der TDS Herstellung notwendig ist. Typischerweise liegt der Wassergehalt eines frisch hergestellten Pflasters unter 2 Gew.-% und ist bevorzugt 1 Gew.-% oder weniger.

In einer besonders bevorzugten erfindungsgemäßen Ausführungsform weist das Transdermale Therapeutische System eine Oberfläche von 10 bis 30 cm², bevorzugt 5 bis 20 cm² auf. Es erübrigt sich zu sagen, dass ein TDS mit einer Oberfläche von zum Beispiel 20 cm² pharmakologisch äquivalent zu zwei 10 cm² Pflastern oder vier 5 cm² Pflastern, die den gleichen Medikamentengehalt pro cm² aufweisen, ist und mit denen ausgetauscht werden kann. Folglich sind die in dieser Anmeldung aufgeführten Oberflächen so zu verstehen, dass sie sich auf die gesamte Oberfläche aller TDS beziehen, die gleichzeitig bei einem Patienten angewendet werden.

Das Zur-Verfügung-Stellen und Auftragen einer oder mehrerer erfindungsgemäßer Transdermaler Therapeutischer Systeme hat den pharmakologischen Vorteil gegenüber einer oralen Therapie, dass der zuständige Arzt die optimale Dosis für den Patienten verhältnismäßig schnell, individuell und genau titrieren kann, z.B. indem einfach die Zahl oder Größe der Pflaster, die dem Patienten gegeben werden, erhöht werden.

Wenn ein Sieben-Tage-Pflaster gewünscht wird, sind höhere Medikamentengehalte im allgemeinen notwendig. Es wurde festgestellt, dass ein Rotigotingehalt im Bereich von ungefähr 0,4 bis 0,5 Gew.-% besonders vorteilhaft ist, da er optimal das in dem TTS enthaltene Medikament nutzt, d.h. nach der Verabreichung ist die restliche Medikamentenmenge in dem TTS nur sehr niedrig. Die Dosis, die unter Verwendung eines solchen TTS verabreicht wird, beträgt gewöhnlich 50 % der Medikamentenmenge, die ursprünglich in dem TTS enthalten ist, oder mehr und kann so hoch wie 80 bis 90 % sein.

Die Tatsache, daß das erfindungsgemäß beschriebene Transdermale Therapeutische System auf Silikonbasis eine signifikante therapeutische Wirkung gegenüber Symptomen des Restless Leg Syndroms sogar bei Oberflächen von 10 bis 20 cm² und insbesondere bei solchen von weniger als 10 cm² und niedrigen Medikamentengehalten von ungefähr 0,4 bis 0,5 mg/cm², inbesondere 0,45 g/cm² bewirkt, muß als weiterer Nutzen der Erfindung angesehen werden.

Das Transdermale Therapeutische System, das erfindungsgemäss verwendet wird, ist vorzugsweise ein Pflaster mit einer kontinuierlich haftenden Matrix, die mindestens in ihrem zentralen Teil das Medikament enthält. Jedoch sind auch transdermale Äquivalente solcher Pflaster erfindungsgemäss umfasst, d.h. eine Ausführungsform, bei der sich das Medikament in einer inerten, aber nicht haftenden Silikonmatrix im zentralen Teil des TTS befindet und ein haftender Teil entlang der Pflasterenden verläuft.

Außerdem betrifft die Erfindung ein Verfahren zur Behandlung der RLS-Krankheit, indem auf einen Patienten ein Transdermales Therapeutisches System mit einer Oberfläche von 5 bis 20 cm² appliziert wird.

Die Erfindung und die beste Ausführungsform werden nachfolgend detaillierter beschrieben.

### Ausführungsbeispiel 4

Ein Transdermales Therapeutisches System unter Verwendung einer Kombination von auf Druck empfindlichen Silikonhaftmitteln wurde wie folgt hergestellt.

(-)-5,6,7,8-Tetrahydro-6-[propyl[2-(2-thienyl)ethyl]amino]-1-naphthalenol-Hydrochlorid (Rotigotinhydrochlorid, 150 g) wurde zu einer Lösung aus 17,05 g NaOH in 218 g Ethanol (96 %) gegeben. Die resultierende Mischung wurde ungefähr 10 Minuten gerührt. Anschließend wurden 23,7 g Natriumphosphatpufferlösung (8,35 g Na₂HPO₄x2H₂O und 16,07 g NaH₂PO₄x2H₂O in 90,3 g Wasser) zugegeben. Nicht löslich oder ausgefällte Stoffe wurden aus der Mischung durch Filtration getrennt. Das Filtrat wurde mit 60,4 g Ethanol (96 %) gewaschen, um eine Teilchen-freie Ethanollösung von Rotigotin in der Form der freien Base zu erhalten.

Die Lösung aus Rotigotin in Form der freien Base (346,4 g) in Ethanol (35 % G/G) wurde mit 36,2 g Ethanol (96 %) gemischt. Die resultierende Lösung wurde mit 109 g einer Ethanollösung, die 25 Gew.-% Polyvinylpyrrolidon (KOLLIDON^{®} 90F) enthielt, 0,077 Gew.-% flüssiger Natriumbisulfitlösung (10 Gew.-%), 0,25 Gew.-% Ascorbylpalmitat und 0,63 Gew.-% DL-alpha-Tocopherol bis zur Homogenität gemischt. Zu der Mischung wurden 817,2 g eines gegenüber Amin beständigen, stark haftenden Silikonhaftmittels (BIO-PSA^{®} Q7-4301, hergestellt von Dow Corning) (74 Gew.-% Lösung in Heptan), 851,8 g eines gegenüber Amin beständigen Silikonhaftmittels von mittlerem Haftvermögen (BIO-PSA^{®} Q7-4201, hergestellt von Dow Corning) (71 Gew.-% Lösung in Heptan) und 205,8 g Petrolether (Heptan) zugegeben und alle Komponenten bis zum Erhalt einer homogenen Dispersion gerührt.

Die Dispersion wurde auf einen geeigneten Abgabeüberzug aus Polyester (SCOTCHPAK^{®} 1022) mit einem geeigneten Arztmesser aufgebracht, und die Lösungsmittel wurden kontinuierlich in einem Trockenofen bei Temperaturen bis zu 80°C ungefähr 30 Minuten entfernt und so eine Medikament-enthaltende haftende Matrix mit einem Überzugsgewicht von 50 g/m² erhalten. Der getrocknete Matrixfilm wurde mit einer Verstärkungsfolie vom Polyestertyp (SCOTCHPAK^{®} 1109) laminiert. Die einzelnen Pflaster wurden aus den vollständigen Laminaten in den gewünschten Größen (z.B. 5 cm², 10 cm², 20 cm², 30 cm²) ausgestanzt und in Beuteln unter Stickstoff-Fluss verschlossen.

Die folgende Tabelle zeigt die Zusammensetzung in mg/20 cm² eines erfindungsgemäßen transdermalen therapeutischen Systems, das eine Kombination aus PSA vom Silikontyp enthält.

| Komponenten der Zusammensetzung | Menge (mg) |
|---|---|
| Rotigotinbase | 9,00 |
| Polyvinylpyrrolidon | 2,00 |
| Silikon BIO-PSA^{®} Q7-4301 | 44,47 |
| Silikon BIO-PSA^{®} Q7-4201 | 44,46 |
| Ascorbylpalmitat | 0,02 |
| DL-Alpha-Tocopherol | 0,05 |
| Natriummetabisulfit | 0,0006 |
| Überzugsgewicht der Matrix | 50 g/m² |

### Klinische Versuche

Das nach Ausführungsbeispiel 4 hergestellte Rotogotin-TDS wurde in placebokontrollierten, doppelblinden, randomisierten (zufallsgesteuerten) klinischen Untersuchungen an mehreren Zentren untersucht und umfaßte als dreiarmige Parallelgruppen aufweisende Studie 63 Patienten, die an mittleren bis sehr schweren idiopathischen Erkrankungen des Restless Leg Syndroms litten.

Das Durchschnittsalter der Patienten war 58,3 Jahre. Die Randomisierung in den drei Behandlungsgruppen im Hinblick auf Geschlecht, Alter und Schwere früherer Erkrankungen war angemessen ausgeglichen.

Nach langsamer und vollständiger Beendigung der bisherigen Therapie mit L-Dopa und einer Therapiepause (washout) von 7 ± 4 Tagen wurden die Patienten mit Rotigotin-TDS behandelt.

Über die Behandlungsdauer von acht (8) Tagen wurden die Patienten aus einer Gruppe mit 5 cm² TDS und die Patienten aus einer weiteren Gruppe mit 10 cm² TDS behandelt. Vergleichsweise wurden die Patienten aus der Placebogruppe mit Placebo-TDS behandelt. Alle Gruppen erhielten jeweils 4 Pflaster als Kombination von Verum- und Placebopflastern. So erhielt die erste Gruppe eine Kombination aus zwei 2,5 cm² Verum-Pflastern und zwei Placebopflaster und die zweite Gruppe vier 2,5 cm² Verum-Pflaster. Die Placebogruppe erhielt vier Placebopflaster. Ein 2,5 cm² TDS wies eine Rotigotinmenge von 1,125 mg auf. 5 cm² enthielten daher eine Rotigotinmenge von 2,25 mg, 10 cm² TDS eine solche von 4,5 mg. Innerhalb von 24 Std. wurden aus den jeweiligen vorgenannten wirkstoffhaltigen TDS ungefähr 50 % des Wirkstoffes (apparente Dosis) Rotigotin an den einzelnen Patienten abgegeben.

Es stellte sich heraus, daß eine wirksame Linderung der Symptome des Restless Leg Syndroms bei Patienten, die an dieser Krankheit leiden, bereits nach einer (1) Woche erreicht wurde. Die Patienten erhielten zu diesem Zeitpunkt keine anderen RLS-wirksamen Medikamente.

Als Ergebnis der präspezifizierten primären Wirksamkeit änderten sich die Aktivitäten des täglichen Lebens und die Motorik nach dem allgemein akzeptierten International Restless Leg Syndrome Study Group (IRLSSG) Rating Scale zwischen dem Ausgangswert und der letzten Behandlungsauswertung (8. Tag).

Der IRLSSG mißt anhand von 10 Fragen die folgenden klinischen Parameter in den Restless-Leg Patienten und stuft sie ein.
1. Wie stark würden Sie im Großen und Ganzen die RLS-Beschwerden in Ihren Beinen oder Armen einschätzen?
   0 = nicht vorhanden = keine Symptome
   1 = leicht
   2 = mäßig
   3 = ziemlich
   4 = sehr schwere Symptome
2. Wie stark würden Sie im Großen und Ganzen Ihren Drang einschätzen, sich wegen Ihrer RLS-Beschwerden bewegen zu müssen?
   0 = nicht vorhanden
   1 = leicht
   2 = mäßig
   3 = ziemlich
   4 = sehr
3. Wie sehr wurden Ihre RLS-Beschwerden in Armen und Beinen im Großen und Ganzen durch Bewegung gelindert?
   0 = es mußten keine RLS-Beschwerden gelindert werden
   1 = vollständig oder fast vollständig
   2 = mäßig
   3 = ein wenig
   4 = überhaupt nicht
4. Wie sehr wurde Ihr Schlaf durch Ihre RLS-Beschwerden gestört?
   0 = überhaupt nicht
   1 = leicht
   2 = mäßig
   3 = ziemlich
   4 = sehr
5. Wie müde oder schläfrig waren Sie tagsüber wegen Ihrer RLS-Beschwerden?
   0 = überhaupt nicht
   1 = ein wenig
   2 = mäßig
   3 = ziemlich
   4 = sehr
6. Wie stark waren Ihre RLS-Beschwerden insgesamt?
   0 = nicht vorhanden
   1 = leicht
   2 = mäßig
   3 = ziemlich
   4 = sehr
7. Wie oft sind Ihre RLS-Beschwerden aufgetreten?
   0 = überhaupt nicht
   1 = von Zeit zu Zeit (das heißt, an 1 Tag in den letzten 7 Tagen
   2 = manchmal (das heißt, an 2 bis 3 Tagen in den letzten 7 Tagen)
   3 = oft (das heißt, an 4 bis 5 Tagen in den letzten 7 Tagen)
   4 = sehr oft (das heißt, an 6 bis 7 Tagen in den letzten 7 Tagen)
8. Wenn Sie RLS-Beschwerden hatten, wie stark waren diese durchschnittlich?
   0 = nicht vorhanden
   1 = leicht (das heißt, an weniger als 1 Stunde an einem 24-Stundentag)
   2 = mäßig (das heißt, an 1 bis 3 Stunden an einem 24-Stundentag)
   3 = ziemlich (das heißt, an 3 bis 8 Stunden an einem 24-Stundentag)
   4 = sehr (das heißt, an 8 Stunden oder mehr an einem 24-Stundentag)
9. Wie sehr haben sich im Großen und Ganzen Ihre RLS-Beschwerden auf Ihre Fähigkeit ausgewirkt, Ihren täglichen Aktivitäten nachzugehen, z.B. ein zufriedenstellendes Familien-, Privat-, Schul- oder Arbeitsleben zu führen?
   0 = überhaupt nicht
   1 = leicht
   2 = mäßig
   3 = ziemlich
   4 = sehr
10. Wie stark haben Ihre RLS-Beschwerden Ihre Stimmung beeinträchtigt, waren Sie z.B. wütend, niedergeschlagen, traurig, ängstlich oder gereizt?
   0 = überhaupt nicht
   1 = leicht
   2 = mäßig
   3 = ziemlich
   4 = sehr

Die IRLSSG-Gesamtbewertung wird aus den einzelnen Werten wie folgt bestimmt:

Zunächst wird ein Ausgangswert für jeden an der Studie teilnehmenden Patienten bestimmt. Und zwar werden die einzelnen Parameterwerte des IRLSSG am Tag 0, d.h. vor der Behandlung addiert. Die IRLSSG-Werte im Behandlungsverlauf werden dann mit diesem Ausgangswert verglichen und Änderungen im Verhältnis zum Ausgangswert werden registriert. Schließlich wird die durchschnittliche Verbesserung des IRLSSG am Tag 8 im Verhältnis zum Ausgangswert bestimmt, indem der Durchschnitt von allen Versuchspersonen genommen wird. Der resultierende Wert wird als FAS-(vollständiger Analysesatz) randomisierte durchschnittliche Änderung vom Ausgangswert der IRLSSG Gesamtwertung bezeichnet. Der Ausdruck "randomisiert" weist auf die Tatsache hin, daß die Patienten hinsichtlich der unterschiedlichen vorbestimmten Dosierungen im Voraus doppelblind randomisiert wurden.

Von Patienten, die an der Krankheit des Restless Leg Syndroms leiden, ist bekannt, daß sie eine verhältnismäßig starke Placebowirkung erfahren, d.h. sogar bei einer Placebo-Behandlung verbessern sich gewissermaßen die IRLSSG-Werte der Restless Leg-Patienten. Es ist daher wichtig, jede Wirkung der Medikamenten-Behandlung mit der Bewertung der IRLSSG Verbesserung, die bei einer Placebobehandlung über einen gleichen Zeitraum erreicht wird, zu vergleichen. Die endgültige Beurteilung der Verbesserung erfolgt daher im Verhältnis zur Wirkung einer Placebobehandlung über den gleichen Zeitraum.

### Ergebnisse

Es trat eine signifikante, Dosis-abhängige Verbesserung der IRLSSG-Werte zwischen dem Ausgangswert und 8 Tagen nach dem Aufbringen eines erfindungsgemäßen TDS auf. Im Vergleich zur Placebogruppe zeigte insbesondere die Gruppe, die mit einem eine Rotigotinmenge von 4,5 mg (apparente Dosis 2,25 mg) aufweisenden TDS behandelt wurde, therapeutisch besonders günstige IRLSSG-Werte. Dieses Ergebnis kann der folgenden Tabelle entnommen werden.

| Größe des Pflasters (TDS) | Rotigotinmenge | Reduktion des durchschnittlichen IRLSSG-Wertes gegenüber der Placebo-Behandlung am 8. Tag | p (einseitig) |
|---|---|---|---|
| 5 cm² | 2,25 mg | 3.6 | 0.09 |
| 10 cm² | 4,5 mg | 6.3 | 0.04 |

Der Wert, der als "p" in der obigen Tabelle angezeigt ist, stellt den einseitigen p-Wert dar, der durch statistische Beurteilung der Versuchsdaten erhalten wurde.

Am Ende der achttägigen Behandlung berichteten beide Patientengruppen, daß nahezu alle subjektiven Symptome wie Kribbeln, Krämpfe, Schmerzen in den Beinen, die Ruhelosigkeit der Beine während der Nacht, die Ein- und Durchschlafstörungen nicht mehr vorhanden oder auf ein tolerables Minimum reduziert waren, so daß ihre tägliche Lebensqualität nicht mehr negativ beeinflußt war.

Die Patienten berichteten weiterhin, daß bei ihnen in Abhängigkeit der verabreichten Dosis des Wirkstoffes Rotigotin nur eine sehr schwache oder gar keine Tagesmüdigkeit, keine Übelkeit, Schwindel, Erbrechen oder Schlaflosigkeit etc. ausgelöst wurden.

Rotigotin wurde, wenn unter Verwendung des erfindungsgemäßen TDS verabreicht, im allgemeinen gut vertragen.

Hautreaktionen an der Auftragungsstelle waren im allgemeinen sehr schwach.

### Schlußfolgerungen

Die obigen Ergebnisse zeigen zum ersten Mal in einer doppelblinden placebokontrollierten Studie, daß ein Dopamin-Agonist (Rotigotin), der transdermal einmal täglich verabreicht wurde, bei Patienten mit Restless Legs Syndrom in mittleren bis schweren Stadien eine deutliche klinische Verbesserung zeigt und gut toleriert wird. Besonders bei Patienten, bei denen die RLS-Symptome verstärkt tagsüber auftraten, war ein Vorteil dieser Medikation festzustellen.

Ein solches Ergebnis konnte mit oral verabreichten Medikamenten bei Monotherapie bisher nicht erreicht werden, wobei eine Verbesserung des IRLSSG-Wertes von 2 im Vergleich zum Placebo schon als Erfolg angesehen werden kann. Eine Verbesserung von über 3 bzw. 6 oder mehr Einheiten bedeutet daher einen noch größeren therapeutischen Fortschritt und ist daher erfindungsgemäß bevorzugt.

### Langzeituntersuchung

Unter Beibehaltung des vorgenannten klinischen Untersuchungsmodells wurde eine Langzeituntersuchung über vier (4) Monate bzw. 120 Tage durchgeführt.

Es wurden wieder nach dem Ausführungsbeispiel 4 hergestellte Rotigotin-TDS verwendet.

Die Untersuchung wurde placebokontrolliert, doppelblind und randomisiert (zufallsgesteuert) als dreiarmige ParallelGruppen aufweisende Studie mit zwölf (12) Patienten, die an mittleren bis sehr schweren idiopathischen Erkrankungen des Restless Leg Syndroms litten, durchgeführt.

Das Durchschnittsalter der Patienten war 60 Jahre. Die Randomisierung in den drei Behandlungsgruppen im Hinblick auf Geschlecht, Alter und Schwere früherer Erkrankungen war angemessen ausgeglichen.

Nach langsamer und vollständiger Beendigung der bisherigen Therapie mit L-Dopa und einer Therapiepause (washout) von 8 ± 4 Tagen wurden die Patienten mit Rotigotin-TDS behandelt.

Über die Behandlungsdauer von vier (4) Monaten wurden die Patienten aus einer Gruppe mit 5 cm² TDS, die Patienten aus einer anderen Gruppe mit 10 cm² TDS und vergleichsweise die Patienten aus der Placebogruppe mit Placebo-TDS behandelt. 5 cm² TDS wiesen eine Rotigotinmenge von 2,25 mg, 10 cm² TDS eine solche von 4,5 mg auf. Innerhalb von 24 Std. wurden aus den jeweiligen vorgenannten wirkstoffhaltigen TDS 50 % des Wirkstoffes (apparente Dosis) Rotigotin an den einzelnen Patienten abgegeben.

Es stellte sich heraus, daß eine wirksame Linderung der Symptome des Restless Leg Syndroms bei Patienten, die an dieser Krankheit leiden, bereits nach einer (1) Woche erreicht wurde. Die Patienten erhielten zu diesem Zeitpunkt keine anderen RLS-wirksamen Medikamente.

Als Ergebnis der präspezifizierten primären Wirksamkeit änderten sich die Aktivitäten des täglichen Lebens und die Motorik nach dem allgemein akzeptierten International Restless Leg Syndrome Study Group (IRLSSG) Rating Scale zwischen dem Ausgangswert und der letzten Behandlungsauswertung (120. Tag).

Der IRLSSG mißt - wie oben beschrieben - anhand von 10 Fragen die klinischen Parameter in den Restless-Leg Patienten und stuft sie ein.

### Ergebnisse

Es trat eine signifikante, Dosis-abhängige Verbesserung der IRLSSG-Werte zwischen dem Ausgangswert und 120 Tagen nach dem Aufbringen eines erfindungsgemäßen TDS auf. Im Vergleich zur Placebogruppe zeigte insbesondere die Gruppe, die mit einem eine Rotigotinmenge von 4,5 mg (apparente Dosis 2,25 mg) aufweisenden TDS behandelt wurde, therapeutisch besonders günstige IRLSSG-Werte.

Am Ende der 120-tägigen Behandlung berichteten beide Patientengruppen, daß alle subjektiven Symptome, die Ein- und Durchschlafstörungen nicht mehr vorhanden oder auf ein Minimum reduziert waren, so daß ihre tägliche Lebensqualität nicht mehr negativ beeinflußt war. Eine Augmentation war nicht signifikant. Die IRLSSG-Werte betrugen in der Gruppe, die mit einem TDS mit 2,25 mg Rotigotinmenge behandelt wurden 12,8, in derjenigen, die mit einem TDS mit 4,5 mg Rotigotinmenge behandelt wurden 15,7.

Die Patienten berichteten weiterhin, daß bei ihnen in Abhängigkeit der verabreichten Dosis des Wirkstoffes Rotigotin über die Behandlungsdauer nur eine sehr schwache oder gar keine Tagesmüdigkeit, keine Übelkeit, Schwindel, Erbrechen oder Schlaflosigkeit etc. ausgelöst wurden.

Rotigotin wurde, wenn unter Verwendung des erfindungsgemäßen TDS verabreicht, im allgemeinen gut vertragen.

Hautreaktionen an der Auftragungsstelle waren im allgemeinen sehr schwach. Wo erforderlich, wurde an einer anderen Stelle der Haut ein TDS aufgetragen. Die frühere Applikationsstelle erholte sich rasch und konnte für eine weitere Behandlung herangezogen werden.

### Schlußfolgerungen

Die obigen Ergebnisse zeigen zum ersten Mal in einer doppelblinden placebokontrollierten Langzeit-Studie, daß ein Dopamin-Agonist (Rotigotin), der transdermal einmal täglich verabreicht wurde, eine relevante klinische Verbesserung bewirkt bei guter Tolerierung und Sicherheit bei Patienten mit der Krankheit des Restless Leg Syndroms im mittleren und schweren Stadium.

Ein solches Ergebnis konnte mit oral verabreichten Medikamenten bei Monotherapie bisher nicht erreicht werden, wobei eine Verbesserung des IRLSSG-Wertes von 2 im Vergleich zum Placebo schon als Erfolg angesehen werden kann. Eine Verbesserung von 10 oder mehr Einheiten bedeutet daher einen noch größeren therapeutischen Fortschritt und ist daher erfindungsgemäß bevorzugt.

Insbesondere zeigt die Studie die Effektivität der erfindungsgemäßen Darreichungsform zur Linderung der Krankheit bei RLS-Patienten, bei denen eine Steigerung der RLS-Beschwerden aufgrund früherer, anderer Medikation zu beobachten war.

## Patentansprüche

1. Verwendung von (-)-5,6,7,8-Tetrahydro-6-[propyl[2-(2-thienyl)ethyl]amino]-1-naphtol zur Herstellung eines Arzneimittels zur transepikutanen Behandlung des Restless Leg Syndroms.

2. Verwendung nach Anspruch 1, **dadurch gekennzeichnet, dass** das Arzneimittel geeignet ist, 0,5 bis 10 mg des Wirkstoffes pro Tag zu applizieren.

3. Verwendung nach Anspruch 1 oder 2, wobei das Arzneimittel ein Transdermales Therapeutisches System (TDS) ist, das eine gegenüber den Inhaltsstoffen der Matrix inerte Rückschicht, eine (-)-5,6,7,8-Tetrahydro-6-[propyl[2-(2-thienyl)ethyl]amino]-1-naphtol enthaltende, selbstklebende Matrixschicht und eine vor Gebrauch zu entfernende Schutzfolie aufweist, **dadurch gekennzeichnet, dass** die Matrixschicht
a) als Basis einen nicht-wäßrigen Polymerkleber auf Acrylat- bzw. Silikonbasis enthält,
b) eine Löslichkeit für die freie Base (-)-5,6,7,8-Tetrahydro-6-[propyl[2-(2-thienyl)ethyl]amino]-1-naphtol von ≥ 5 % (g/g) besitzt, und
c) die freie Base (-)-5,6,7,8-Tetrahydro-6-[propyl[2-(2-thienyl)ethyl]amino]-1-naphtol in einer wirksamen Menge enthält.

4. Verwendung nach Anspruch 3, **dadurch gekennzeichnet, dass** die Matrix < 0,5 % (g/g) anorganische Silikatpartikel enthält.

5. Verwendung nach Anspruch 3, **dadurch gekennzeichnet, dass** die Matrix < 0,05 % (g/g) anorganische Silikatpartikel enthält.

6. Verwendung nach Anspruch 3, in dem der Polymerkleber auf Acrylatbasis wenigstens zwei der folgenden Monomere enthält:
Acrylsäure, Acrylamid, Hexylacrylat, 2-Ethylhexyl-acrylat, Hydroxyethylacrylat, Octylacrylat, Butylacrylat, Methylacrylat, Glycidylacrylat, Methacrylatsäure, Methacrylamid, Hexylmethylacrylat, 2-Ethylhexylmethacrylat, Octylmethacrylat, Methylmethacrylat, Glycidylmethacrylat, Vinylacetat oder Vinylpyrrolidon.

7. Verwendung nach Anspruch 3, in welchem der Polymerkleber auf Silikonbasis Zusatzstoffe zur Verbesserung der Löslichkeit von (-)-5,6,7,8-Tetrahydro-6-[propyl[2-(2-thienyl)ethyl]amino]-1-naphtol in Form von hydrophilen Polymeren oder Glycerin oder Glycerinderivaten enthält.

8. Verwendung nach Anspruch 6 oder 7, in welchem (-)-5,6,7,8-Tetrahydro-6-[propyl[2-(2-thienyl)ethyl]amino]-1-naphtol in dem Polymerkleber auf Acrylatbasis in einer Konzentration von 10 bis 35 % [g/g] oder in dem Polymerkleber auf Silikonbasis in einer Konzentration von 5 bis 40 % [g/g] enthalten ist.

9. Verwendung nach Anspruch 8, wobei das TDS die Permeation von (-)-5,6,7,8-Tetrahydro-6-[propyl[2-(2-thienyl)ethyl]amino]-1-naphtol in die menschliche Haut verbessernde Substanzen ausgewählt aus der Gruppe der Fettalkohole, Fettsäuren, Fettsäureester, Fettsäureamide, Glycerin oder seinen Derivaten, N-Methylpyrrolidon, Terpenen oder Terpenderivaten, enthält.

10. Verwendung nach Anspruch 9, in welcher die permeationsfördernde Substanz Ölsäure oder Oleylalkohol ist.

11. Verwendung nach Anspruch 7, in welchem das hydrophile Polymer Polyvinylpyrrolidon, ein Copolymer von Vinylpyrrolidon und Vinylacetat, Polyethylenglykol, Polypropylenglykol oder ein Copolymer von Ethylen und Vinylacetat ist.

12. Verwendung nach Anspruch 11, in dem das hydrophile Polymer lösliches Polyvinylpyrrolidon ist und in einer Konzentration von 1,5-5 % (g/g) in der wirkstoffhaltigen Matrixschicht enthalten ist.

13. Verwendung nach Anspruch 3, wobei das TDS eine Mischung aus mindestens einem stark klebendem Silikonhaftmittel und mindestens einem Silikonhaftmittel mit mittlerem Klebevermögen umfasst.

14. Verwendung nach Anspruch 13, wobei das TDS weiter einen Lösungsvermittler umfasst.

15. Verwendung nach Anspruch 14, wobei der Lösungsvermittler lösliches Polyvinylpyrrolidon ist.

16. Verwendung nach einem der Ansprüche 13 bis 15, wobei das TDS weniger als 1 Gew.-% anorganischer Silikate enthält.

17. Verwendung nach Anspruch 16, wobei das TDS frei von anorganischen Silikaten ist.

18. Verwendung nach Anspruch 3, wobei das TDS eine Größe von 5 bis 20 cm² aufweist und 0,4 bis 0,5 mg/cm² Rotigotin als aktiven Bestandteil in seiner Matrix enthält, die hauptsächlich eine Mischung aus mindestens zwei gegenüber Amin beständigen Silikonhaftmitteln umfasst.

19. Verwendung eines transdermalen therapeutischen Systems auf Silikonbasis mit einer Oberfläche von 5 bis 20 cm² und enthaltend 0,1 bis 3,15 mg/cm² Rotigotin als aktiven Bestandteil zur Herstellung eines Anti-Restless Leg Syndrom Medikaments, das bei einem humanen Restless Leg Syndrom-Patienten gegenüber einer Placebobehandlung gemäß dem International Restless Leg Syndrome Study Group Rating Scale (IRLSSG) eine Verbesserung von 2 Einheiten oder mehr nach mindestens achttägigem Auftragen bewirkt.

## Claims

1. Use of (-)-5,6,7,8-tetrahydro-6-[propyl[2-(2-thienyl)ethyl]amino]-1-naphthol for the production of a medicament for the transepicutaneous treatment of Restless Leg Syndrome.

2. Use according to claim 1, **characterised in that** the medicament is suitable for applying 0.5 to 10 mg of active substance per day.

3. Use according to claim 1 or 2, wherein the medicament is a transdermal therapeutic system (TDS) comprising a backing layer that is inert with respect to the components of the matrix, a self-adhesive matrix layer containing (-)-5,6,7,8-tetrahydro-6-[propyl[2-(2-thienyl)ethyl]amino]-1-naphthol, and a protective film to be removed before use, **characterised in that** said matrix layer
a) contains, as the base, a non-aqueous acrylate or silicone-based polymer adhesive,
b) has a solubility of ≥ 5 % (g/g) for the free base (-)-5,6,7,8-tetrahydro-6-[propyl[2-(2-thienyl)ethyl]amino]-1-naphthol, and
c) contains the free base (-)-5,6,7,8-tetrahydro-6-[propyl[2-(2-thienyl)ethyl]amino]-1-naphthol in an effective amount.

4. Use according to claim 3, **characterised in that** the matrix contains < 0.5 % (g/g) of inorganic silicate particles.

5. Use according to claim 3, **characterised in that** the matrix contains < 0.05 % (g/g) of inorganic silicate particles.

6. Use according to claim 3, in which the acrylate-based polymer adhesive comprises at least two of the following monomers:
acrylic acid, acrylamide, hexyl acrylate, 2-ethyl hexyl acrylate, hydroxyethyl acrylate, octyl acrylate, butyl acrylate, methyl acrylate, glycidyl acrylate, methacrylate acid, methacrylamide, hexyl methyl acrylate, 2-ethyl hexyl methacrylate, octyl methacrylate, methyl methacrylate, glycidyl methacrylate, vinyl acetate or vinyl pyrrolidone.

7. Use according to claim 3, in which the silicone-based polymer adhesive contains additives for improving the solubility of (-)-5,6,7,8-tetrahydro-6-[propyl[2-(2-thienyl)ethyl]amino]-1-naphthol in the form of hydrophilic polymers or glycerine or glycerine derivatives.

8. Use according to claim 6 or 7, in which (-)-5,6,7,8-tetrahydro-6-[propyl[2-(2-thienyl)ethyl]amino]-1-naphthol is contained in the acrylate-based polymer adhesive in a concentration of 10 to 35 % [g/g] or in the silicone-based polymer adhesive in a concentration of 5 to 40 % [g/g].

9. Use according to claim 8, wherein the TDS contains substances that improve the permeation of (-)-5,6,7,8-tetrahydro-6-[propyl[2-(2-thienyl)ethyl]amino]-1-naphthol into human skin, selected from the group of fatty alcohols, fatty acids, fatty acid esters, fatty acid amides, glycerine or derivatives thereof, N-methyl pyrrolidone, terpenes or terpene derivatives.

10. Use according to claim 9, in which the permeation-promoting substance is oleic acid or oleyl alcohol.

11. Use according to claim 7, in which the hydrophilic polymer is polyvinyl pyrrolidone, a copolymer of vinyl pyrrolidone and vinyl acetate, polyethylene glycol, polypropylene glycol or a copolymer of ethylene and vinyl acetate.

12. Use according to claim 11, in which the hydrophilic polymer is soluble polyvinyl pyrrolidone and is contained in a concentration of 1.5 to 5 % (g/g) in the active substance-containing matrix layer.

13. Use according to claim 3, wherein the TDS comprises a mixture of at least one high tack silicone adhesive and at least one medium tack silicone adhesive.

14. Use according to claim 13, wherein the TDS further comprises a solubiliser.

15. Use according to claim 14, wherein the solubiliser is (soluble) polyvinyl pyrrolidone.

16. Use according to one of claims 13 to 15, wherein the TDS contains less than 1 % by weight of inorganic silicates.

17. Use according to claim 16, wherein the TDS is free from inorganic silicates.

18. Use according to claim 3, wherein the TDS has a size of 5 to 20 cm² and contains 0.4 to 0.5 mg/cm² of rotigotine as the active component in its matrix, which primarily comprises a mixture of at least two amine-resistant silicone adhesives.

19. Use of a silicone-based transdermal therapeutic system having a surface area of 5 to 20 cm² and containing 0.1 to 3.15 mg/cm² of rotigotine as the active component for the production of an anti-Restless Leg Syndrome medicament which, in accordance with the International Restless Leg Syndrome Study Group Rating Scale (IRLSSG), leads to an improvement of 2 units or more in human patients suffering from Restless Leg Syndrome as compared to a placebo treatment after at least eight days of application.

## Revendications

1. Utilisation du (-)-5,6,7,8-tétrahydro-6-[propyl[2-(2-thiényl)éthyl]amino]-1-naphtol pour la fabrication d'un médicament destiné au traitement transcutané du syndrome des jambes sans repos.

2. Utilisation selon la revendication 1, ***caractérisée en ce que*** le médicament permet l'administration de 0,5 à 10 mg du principe actif par jour.

3. Utilisation selon la revendication 1 ou 2, dans laquelle le médicament est un système thérapeutique transdermique (STT) possédant une couche de support inerte au contact des composants de la matrice, une couche de matrice autocollante contenant du (-)-5,6,7,8-tétrahydro-6-[propyl[2-(2-thiényl)éthyl]amino]-l-naphtol et un film protecteur à retirer avant utilisation, ***caractérisée en ce que*** la couche de matrice
a) contient comme base un adhésif polymère non aqueux à base d'acrylate ou de silicone,
b) permet une solubilité de la base libre du (-)-5,6,7,8-tétrahydro-6-[propyl[2-(2-thiényl)éthyl]amino]-1-naphtol supérieure ou égale à 5 % (g/g),
c) contient la base libre du (-)-5,6,7,8-tétrahydro-6-[propyl[2-(2-thiényl)éthyl] amino]-1-naphtol en quantité efficace.

4. Utilisation selon la revendication 3, ***caractérisée en ce que*** la matrice contient moins de 0,5 % (g/g) de particules de silicate inorganique.

5. Utilisation selon la revendication 3, ***caractérisée en ce que*** la matrice contient moins de 0,05 % (g/g) de particules de silicate inorganique.

6. Utilisation selon la revendication 3, dans laquelle l'adhésif polymère à base d'acrylate contient au moins deux des monomères suivants : acide acrylique, acrylamide, acrylate d'hexyle, acrylate de 2-éthylhexyle, acrylate d'hydroxyéthyle, acrylate d'octyle, acrylate de butyle, acrylate de méthyle, acrylate de glycidyle, acide méthacrylique, méthacrylamide, acrylate d'hexylméthyle, méthacrylate de 2-éthylhexyle, méthacrylate d'octyle, méthacrylate de méthyle, méthacrylate de glycidyle, acétate de vinyle ou vinylpyrrolidone.

7. Utilisation selon la revendication 3, dans laquelle l'adhésif polymère à base de silicone contient des additifs pour améliorer la solubilité du (-)-5,6,7,8-tétrahydro-6-[propyl [2-(2-thiényl)éthyl]amino]-1-naphtol sous la forme de polymères hydrophiles ou de glycérine ou de dérivés de glycérine.

8. Utilisation selon la revendication 6 ou 7, dans laquelle le (-)-5,6,7,8-tétrahydro-6-[propyl[2-(2-thiényl)éthyl]amino]-1-naphtol est contenu dans l'adhésif polymère à base d'acrylate selon une concentration de 10 à 35 % (g/g) ou dans l'adhésif polymère à base de silicone selon une concentration de 5 à 40 % (g/g).

9. Utilisation selon la revendication 8, dans laquelle le STT contient des substances améliorant la pénétration du (-)-5,6,7,8-tétrahydro-6-[propyl[2-(2-thiényl)éthyl]amino]-1-naphtol à travers la peau humaine, choisies parmi les alcools gras, les acides gras, les esters d'acides gras, les amides d'acides gras, la glycérine ou ses dérivés, la N-méthylpyrrolidone, les terpènes ou les dérivés de terpène.

10. Utilisation selon la revendication 9, dans laquelle la substance favorisant la pénétration est de l'acide oléique ou de l'alcool oléylique.

11. Utilisation selon la revendication 7, dans laquelle le polymère hydrophile est de la polyvinylpyrrolidone, un copolymère de vinylpyrrolidone et d'acétate de vinyle, du polyéthylène-glycol, du polypropylène-glycol ou un copolymère d'éthylène et d'acétate de vinyle.

12. Utilisation selon la revendication 11, dans laquelle le polymère hydrophile est de la polyvinylpyrrolidone soluble et est présent selon une concentration de 1,5 à 5 % (g/g) dans la couche de matrice contenant le principe actif.

13. Utilisation selon la revendication 3, dans laquelle le STT comprend un mélange d'au moins un adhésif au silicone à forte adhérence et d'au moins un adhésif au silicone à adhérence moyenne.

14. Utilisation selon la revendication 13, dans laquelle le STT comprend en outre un activateur de dissolution.

15. Utilisation selon la revendication 14, dans laquelle l'activateur de dissolution est de la polyvinylpyrrolidone soluble.

16. Utilisation selon l'une quelconque des revendications 13 à 15, dans laquelle le STT contient moins de 1 % en poids de silicates inorganiques.

17. Utilisation selon la revendication 16, dans laquelle le STT ne contient pas de silicates inorganiques.

18. Utilisation selon la revendication 3, dans laquelle le STT a une taille de 5 à 20 cm² et contient entre 0,4 et 0, 5 mg/cm² de rotigotine comme principe actif dans sa matrice, qui comprend principalement d'un mélange d'au moins deux adhésifs au silicone résistants aux amines.

19. Utilisation d'un système thérapeutique transdermique à base de silicone ayant une surface de 5 à 20 cm² et contenant de 0,1 à 3,15 mg/cm² de rotigotine comme principe actif pour la fabrication d'un médicament contre le syndrome des jambes sans repos qui permet d'obtenir, chez un patient humain atteint du syndrome des jambes sans repos, une amélioration de 2 unités ou plus selon l'échelle de notation du Groupe international d'étude du syndrome des jambes sans repos (IRLSSG) après au moins huit jours d'application par rapport au traitement par un placebo.
